# EUROPEAN PATENT APPLICATION

(11) **EP 3 758 020 A1**
(43) Date of publication of application: **30.12.2020**
(21) Application number: 19182348.3
(22) Date of filing: 25.06.2019
(51) Int. Cl.: G16H 40/63

(54) **SYSTEMS AND METHODS FOR GENERATING USER-SPECIFIC FEEDBACK**

(71) Applicant: Koninklijke Philips N.V., 5656 AG Eindhoven (NL)
(72) Inventor: HIGGINS, Paul, 5656 AE Eindhoven (NL); SUN, Wen (Winnie), 5656 AE Eindhoven (NL)
(74) Representative: de Haan, Poul Erik

(57) **Abstract**

The invention provides a system for generating user-based feedback for a user. The system includes a user sensor adapted to acquire user data and a feedback management unit. The feedback management unit is adapted to obtain a user profile, the user profile comprising: a user goal; and a user experience level relating to the amount of time the user has been pursuing the user goal. Further, the feedback management unit is adapted to generate a mood prediction based on the acquired user data and select a feedback type and a feedback valance based on the user experience level and the mood prediction. The feedback management unit then generates feedback based on the selected feedback type and feedback valance. The system further includes a user interface adapted to provide the generated feedback to the user.

## Description

### FIELD OF THE INVENTION

The invention relates to the field of generating user-specific feedback, and more specifically to the field of automatically generating and providing user-specific feedback.

### BACKGROUND OF THE INVENTION

Setting goals and receiving feedback on the attainment of goals are fundamental techniques in achieving a behavior change and are frequently implemented as functions in mobile health applications. At the core of lifestyle behavioral changes are three inter-related techniques: self-monitoring of behavior; setting goals; and receiving feedback on performance towards attaining goals. The nature and timing of the feedback, as well as the user's underlying affective state, influences the way the user responds to feedback.

Of these techniques, regular feedback on a pre-set goal is recognized as fundamental in user-directed behavioral changes as it helps to sustain motivation and stimulate continuous effort towards the final goal in the absence of a healthcare provider's direct input. Nevertheless, it is often overlooked that feedback can be more or less effective depending on a variety of factors at the time the feedback is received.

There is therefore a need for a means of generating and providing user-specific feedback to a user in a manner that optimizes the likelihood of success in the user achieving their goal.

### SUMMARY OF THE INVENTION

The invention is defined by the claims.

According to examples in accordance with an aspect of the invention, there is provided a system for generating user-based feedback for a user, the system comprising:
a user sensor adapted to acquire user data;
a feedback management unit, wherein the feedback management unit is adapted to:
   obtain a user profile, the user profile comprising:
      a user goal; and
      a user experience level relating to the amount of time the user has been pursuing the user goal;
   generate a mood prediction based on the acquired user data;
   select a feedback type and a feedback valance based on the user experience level and the mood prediction; and
   generate feedback based on the selected feedback type and feedback valance; and
a user interface adapted to provide the generated feedback to the user.

The invention provides for a means of generating user specific feedback based on a user's mood and the length of time they have been pursuing the goal.

A user's mood is known to affect how receptive they are to a given form of feedback. Further, the time for which they have been pursuing a goal will also effect the reception and efficacy of a given type of feedback.

By tailoring the feedback type and valance based on both the user's mood and the stage of the goal, it is possible to generate feedback that is likely to have the optimal effect on the user's motivation towards pursuing the goal.

The combination of both the user's mood and the length of time that the user has been pursuing the goal provides for a significantly more accurate assessment of the type of feedback and the optimal timing for providing the feedback that would most benefit the user.

In an embodiment, the user sensor is adapted to sense one or more of:
a movement pattern;
a physical activity;
a galvanic skin response;
a heart rate; and
a user interaction with a system.

In this way, a number of different sensor outputs may be used to assess the user's mood.

In an embodiment, the user profile further comprises historic user data.

By providing historic user data, it is possible to assess the performance of a user over time.

In a further embodiment, the feedback management unit is adapted to generate a mood prediction comprises comparing the obtained user data to the historic user data.

In this way, the current data may be compared to the historic user data to generate a more informed decision as to the user's current mood.

In an arrangement, the user profile further comprises a goal performance relating to an attainment of the user goal, and wherein the feedback management unit is further adapted to select the feedback type and the feedback valence based on the goal performance.

In this way, the level of attainment of the user in the pursuit of the goal may be taken into account when selecting the appropriate feedback.

In an embodiment, the user profile further comprises a user self-assessment relating to a user's perception of their performance towards the user goal, and wherein the feedback management unit is further adapted to select feedback type and the feedback valence is further based on the user self-assessment.

In this way, the user's perception of their own performance may be taken into account when selecting the appropriate feedback.

In an embodiment, the feedback type comprises one or more of:
verification feedback; and
informative feedback.

In this way, the amount of detail in the feedback may be adjusted according to how receptive the user will be.

In an embodiment, the feedback valance comprises one or more of:
positive feedback; and
negative feedback.

In this way, the tone of the feedback may be adjusted according to how receptive the user will be.

In an embodiment, the user sensor and the user interface are located within a smart device of the user.

In this way, the system may be integrated into a single smart device.

In a further embodiment, the smart device comprises one or more of:
a smartphone; and
a smart watch.

According to examples in accordance with an aspect of the invention, there is provided a computer-implemented method for generating user specific feedback for a user, the method comprising:
obtaining user data;
obtaining a user profile, the user profile comprising:
   a user goal; and
   a user experience level relating to the amount of time the user has been pursuing the user goal;
generating a mood prediction based on the acquired user data;
selecting a feedback type and a feedback valance based on the user experience level and the mood prediction;
generating feedback based on the selected feedback type and feedback valance; and
providing the generated feedback to the user.

In an embodiment, the user profile further comprises goal performance, and wherein selecting the feedback type and the feedback valence is further based on the goal performance.

In an embodiment, the user profile further comprises a user self-assessment, and wherein selecting the feedback type and the feedback valence is further based on the user self-assessment.

In an embodiment, the method further comprises updating the user profile based on the user data and the selected feedback type and feedback valance.

In this way, the user profile may be continually updated based on the most recent feedback provided to the user.

According to examples in accordance with an aspect of the invention, there is provided a computer program comprising computer program code means which is adapted, when said computer program is run on a computer, to implement the methods described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a system for generating user-based feedback for a user;
Figure 2 shows a schematic representation of a feedback management unit;
Figure 3 shows a schematic representation of an exemplary state and stage detector; and
Figure 4 shows a method for generating user-based feedback for a user.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for generating user-based feedback for a user. The system includes a user sensor adapted to acquire user data and a feedback management unit. The feedback management unit is adapted to obtain a user profile, the user profile comprising: a user goal; and a user experience level relating to the amount of time the user has been pursuing the user goal. Further, the feedback management unit is adapted to generate a mood prediction based on the acquired user data and select a feedback type and a feedback valance based on the user experience level and the mood prediction. The feedback management unit then generates feedback based on the selected feedback type and feedback valance. The system further includes a user interface adapted to provide the generated feedback to the user.

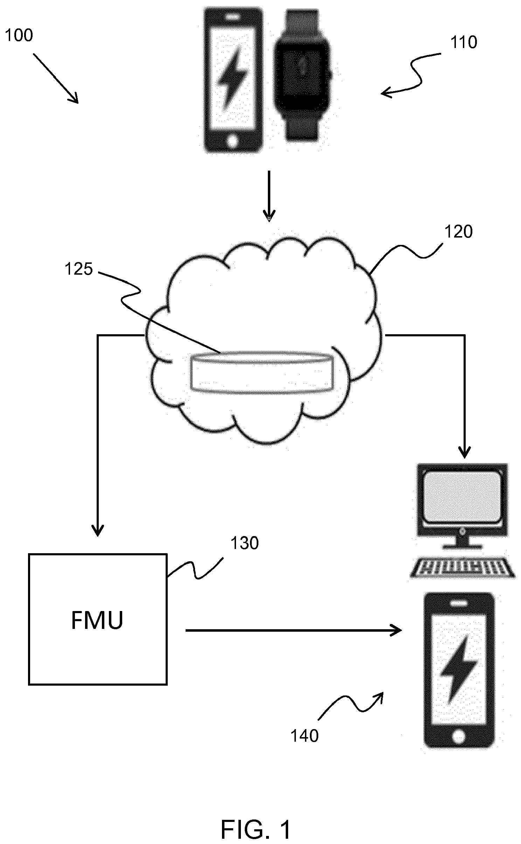

Figure 1 shows a system 100 for generating user-based feedback for a user.

The system 100 includes a user sensor 110 adapted to acquire user data, which may include one or more of: a movement pattern, for example acquired by an accelerometer; a physical activity; a galvanic skin response, for example acquired by an electrode in contact with the user's skin; a heart rate, for example acquired using a heart rate monitor; and a user interaction with a system. The user interaction may, for example, include user data aggregated from a number of calls made by the user, social networking activity level of the user, usage activity level of a given application, the overall screen time, and the like. These measures may be utilized in an assessment of the user's mood. The user sensor 110 may be located within a smart device of the user, such as a smartphone and/or a smart watch. The user sensor may also be located in any suitable device carried on the user's person.

The acquired user data may be transferred from the user sensor to a processing unit 120, which may be a local processing unit or a remote processing unit. A local processing unit may be a processor located within the same device as the user sensor, such as a smartphone. A remote processing unit may be a cloud processing resource that is in communication with the user sensor by a wired/wireless link. In the example shown in Figure 1, the processing unit 120 is a remote processing unit, and more specifically, a cloud processing resource.

The processing unit 120 may aggregate the user data in a memory unit 125, which may then be provided to a feedback management unit 130 and/or a user interface 140.

Put another way, the system 100 operates using sensors from a smart phone or from a multitude of wearable technologies capable of tracking user data relating, for example, to physical activity, movement, physiological function and social interaction. This system may be operated and/or utilized within an existing mobile or electronic health application that provides feedback to a user regarding their progress towards obtaining a goal. Further, the system may take advantage of existing connected platforms and access to the cloud, for example, via WLAN or 4G connections. In the system 100 shown in Figure 1, user data from the user sensor 110 is aggregated on a cloud server and managed for analysis.

The system 100 further comprises a feedback management unit 130 for analyzing the user data and selecting the appropriate feedback to provide to the user.

The feedback management unit 130 is adapted to obtain a user profile, the user profile comprising a user goal and a user experience level relating to the amount of time the user has been pursuing the user goal. The user profile may further comprise historic user data, i.e. user data captured over a period of time. The user goal may be, for example, a dietary goal or an exercise goal.

In other words, the user profile may be maintained and continuously updated with objectively obtained user start date data, relating to the date when the user started pursuing the user goal. Further, the user profile may also include subjective information relating to user self-efficacy, which may be obtained from the user using a digital visual analogue scale (dVAS) and user response efficacy, which may also be captured using a dVAS.

In an example, the user initiating the system and recording three consecutive days of usage may be taken as the start time of the user pursuing the user goal.

The user's past success in the program may also recorded using historic user data. In this case, a rule may be utilized to continuously update the user's profile (for example, weekly) and prompt completion of the dVAS to maintain updated records on both the user self-efficacy and the user response efficacy. Hence, on the basis of the dimensions of: time in program (days); self-efficacy (rating between 0-100); and response efficacy (rating between 0-100), each user may be provided with a continuously updated profile on which to feedback type and feedback valence may be tailored.

The feedback management unit is further adapted to generate a mood prediction based on the acquired user data.

For example, the system 100 may utilize mobile phone or fitness watch sensors as user sensors 110 to generate user data that is processed into a quantitative assessment of mood. User data such as: movement patterns; physical activity; galvanic skin response; heart rate; and user's interactions with the system (for example typing dynamics, frequency and duration of phone conversations, frequency and duration of messaging, social media usage, frequency of app usage and the like) may be analyzed in order to assess the user's mood.

Some combination of the various types of user data may be utilized based on the availability of sensors, and user's willingness to share pertinent data, and reduced into a single quantitative variable. The mood prediction may be assessed on a continuous basis and a record of past and current mood states may be generated and maintained.

The generation of the mood prediction may include comparing the obtained user data to historic user data in order to associate trends in user data with user mood, thereby increasing the accuracy of the mood prediction.

Finally, the feedback management unit 130 selects a feedback type and a feedback valance based on the user experience level and the mood prediction and generates feedback to provide to the user based on the selected feedback type and feedback valance.

The feedback type may include verification feedback and/or informative feedback and the feedback valance may include positive feedback and/or negative feedback.

In some cases, the user profile further comprises a goal performance relating to an attainment of the user goal, and wherein the feedback management unit is further adapted to select the feedback type and the feedback valence based on the goal performance. Further, the user profile may comprise a user self-assessment relating to a user's perception of their performance towards the user goal, and wherein the feedback management unit is further adapted to select feedback type and the feedback valence is further based on the user self-assessment.

Looking to the feedback valance, positive feedback emphasizes a user's success over the user's failures. Conversely, negative feedback highlights unsuccessful outcomes more than successful outcomes. At their most rudimentary, theories of motivation propose that positive feedback leads to a positive mood/affect, while negative feedback leads to a negative mood/affect. Indeed, the mental processing of positive feedback and negative feedback is undertaken using different cognitive mechanisms. Positive feedback is processed by reinforcement learning; whereas, the processing of negative feedback occurs by cognitive information processing.

By extension, negative feedback should not be provided if a user is already in a negative mood as it would not be well received, would not be processed adequately, and perhaps even worsen the user's mood. Nonetheless, it is important to note that the provision of negative feedback is essential in many circumstances.

Positive feedback is important when users are starting out on the pursuit of a new goal. In other words, when a user first starts pursuing a new goal, positive feedback should be favored over negative feedback. Positive feedback may boost outcome expectancy (response efficacy) and self-efficacy, whilst also increasing the user's confidence.

However, positive feedback may be problematic in some cases as it may encourage a sense of complacency, a sense of partial goal attainment, and a relaxation of effort. On the other hand, negative feedback may reduce a user's confidence, particularly if self-efficacy and response efficacy are low. However, negative feedback can be helpful to encourage users at a more advanced stage of goal pursuit and competency in order to increase effort at key stages where behavior change maintenance is essential to goal success.

In addition to the effects of positive and negative feedback, the user experience level (i.e. the amount of time the user has been pursuing the user goal) will affect how feedback will be received. The user experience level may be categorized according to whether a user is highlighting towards a goal or balancing towards a goal.

A user who is highlighting is typically at an early stage of pursuing a new goal. A user that is highlighting tends to be highly focused on the goal and take actions that are consistent with the goal. These actions typically complement each other towards the user goal, for example, a user pursuing a diet goal stops eating fried food and drinking sugary drinks on weekends. This user would be openly expressing commitment to the dietary goal, has a high expectancy for success and is confident. Each action taken further affirms this user's commitment to achieving the goal. According to the transtheoretical model/stages of change model, they are between the preparation and the early-action stages.

In contrast, a user who is balancing is typically at a more advanced stage, i.e. the early excitement and motivation for achieving the goal may have declined and the final goal is not at the forefront of their mind. The user likely rerecognizes the need to attain the goal, but sees each step as building towards attainment of the goal. In this case, the user is typically deep into the program and is progressing towards the goal step-by-step, for example, a user pursuing a diet goal may choose to eat a salad for lunch on Monday knowing that they consumed a fried breakfast on Sunday.

In the balancing category, users are more likely to follow a goal-consistent action if the prior action was not goal-consistent. According to the transtheoretical model, these users will fall within the mid-to-late action and maintenance phases.

Taking the feedback valance and user experience level both into account, the feedback management unit may determine the following. The response to positive feedback may be a boost in motivation and effort when given in a time of highlighting as it enhances the sense commitment to the goal. The response to positive feedback may be a decline in motivation and effort when given in a time of balancing as it suggests sufficient progress and effort. The response to negative feedback may be a decline in motivation and effort when given in in a time of highlighting as it erodes the sense of commitment to the goal. The response to negative feedback may be a boost in motivation and effort when given in a time of balancing as it suggests insufficient progress.

In summary, positive feedback may be more useful when a user is actively establishing their commitment to a goal; whereas, negative feedback may be more useful when an individual needs to increase effort in actively pursuing the goal day-to-day (balancing). In fact, negative feedback processing may be crucial when the user data highlights a gap between a goal and current performance and the user is required to take action to close this gap.

Put another way, a user's sense of commitment may change over time. It is self-evident that a user is actively establishing their commitment (highlighting) at the outset of pursuing a goal, but may also be re-establishing commitment at a later time during a program where a significantly new goal has been set. It follows that a user is less likely to be evaluating their commitment and are more likely to be balancing with detailed progress monitoring as they become more experienced at pursuing the goal. Indeed, users who are more experienced in a given task are more likely to seek negative feedback while those less experienced more likely to seek positive feedback.

As discussed above, the provision of feedback can alter an individual's mood. Moreover, the identification of a user being in a positive or negative mood may be a mechanism through which the feedback management unit 130 may provide feedback to drive motivation and effort. Further, the mood prediction may provide goal progress information.

However, a user's underlying mood at the time the feedback is given (unrelated to the feedback itself) has an influence on the way in which feedback is processed and acted upon. Mood may be considered as a self-regulatory resource to manage information. Furthermore, whether a user attributes their present positive or negative mood to the actual feedback that is given has an important impact on whether the feedback has the necessary effect on effort towards the goal. In a period when an individual is seeking to boost commitment towards a goal (highlighting), an underlying positive affect at the time positive feedback is given is likely to further boost motivation towards the goal. Further, if feedback is given to a user that is highlighting at a time of a negative mood, it is likely to have a sub-optimal influence on goal-directed effort.

Moreover, for a user who is balancing, the importance of negative feedback is paramount and if this negative feedback is given at a time of underlying positive mood, then this user (with more cognitive resources) will be able to process the information contained within the negative feedback better and will be more likely to fully act on the negative feedback provided. Accordingly, by accounting for both the user's mood and their experience level, it is possible to adjust the timing and the valence of the feedback to optimize the user's response and resulting effort towards the goal. Further, as mood is not a simple binary state and there is a continuum of affective states between a very bad mood and very good mood, a continuous quantitative representation of mood may be provided to enhance the accuracy of the feedback management unit.

In addition to the valence of the feedback (positive or negative), feedback type also has a major influence on motivation and effort. Two general categories of feedback type can be delineated: confirmatory or verification feedback; and informative feedback. Confirmatory feedback simply indicates whether an individual is on track or not, i.e. it provides minimal information and is useful at the very early stages of goal pursuit and also at times when cognitive load is high, for example during times of negative mood.

In contrast, informative feedback is much more detailed and may, for example, provide information on the size of the performance gap, why an individual has not achieved the target behavior, and may even provide information on how to make corrective actions. Informative feedback is useful for users that are more advanced in the pursuit of the goal (balancers). However, informative feedback may impose a considerable cognitive load. To ensure adequate processing of highly detailed informative feedback, the feedback may be administered at a time of relative cognitive ease when more cognitive resources are available. Such a time is typically associated with a prevailing positive mood.

Further, the presence of positive mood predicts increased attention to negative information about the user and supports more thorough processing of negative information if that negative information is perceived as useful for the user's goal.

In addition, the feedback management unit may adjust the feedback to account for the user's feelings of self-efficacy. A user's self-efficacy, or belief in their own ability to achieve the goal (confidence), may be considered within the context of the feedback type and valence. Users with low self-efficacy will benefit more from positive feedback than those with already high self-efficacy. Therefore, it may be possible to determine the feedback's valence with knowledge of self-efficacy.

Response efficacy, *i.e.* whether the feedback information is perceived as useful, may have implications for the selection of the feedback valence. When negative feedback is provided during a time of positive affect, it is better processed when the information is perceived to be useful. The user may provide an indication of perceived usefulness of certain feedback aspects in order to select the type and valence of the feedback for future use.

The system 100 further includes a user interface 140 adapted to provide the generated feedback to the user. The user interface may be located within a smart device of the user such as a smartphone and/or a smart watch. The user interface may be located in the same device as the user sensor. The user interface may include an application for handling user interactions, for example, for collecting user self-efficacy or response efficacy ratings.

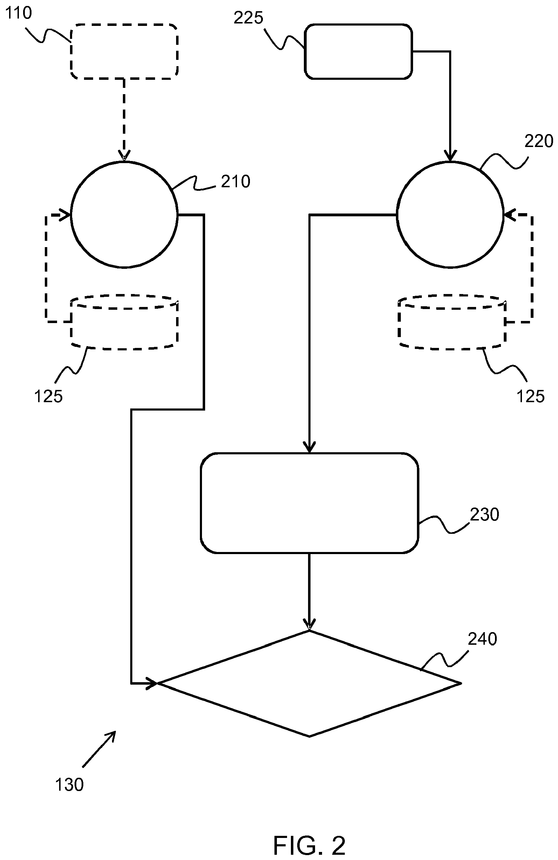

Figure 2 shows a schematic representation of a feedback management unit 130.

The feedback management unit 130 may include a mood predictor unit 210, which is adapted to receive user data from the user sensor 110 (and may also receive historic user data from a memory unit 125) and generate a mood prediction for the user. The user data may comprise a variety of data types as discussed above.

The mood prediction may be generated using the user data as described in one or more of: Likamwa R, Liu Y, Lane ND, Zhong L. MoodScope: Building a Mood Sensor from Smartphone Usage Patterns. MobiSys' 13, June 25-28, 2013, Taipei, Taiwan; Stutz T, et al. Smartphone Based Stress Prediction. F. Ricci et al. (Eds.): UMAP 2015, LNCS 9146, pp. 240-251. DOI: 10.1007/978-3-319-20267-9 20; and Cao B, Zheng L, Zhang C, et al. DeepMood: Modeling mobile phone typing dynamics for mood detection. Association for Computing Machinery 2017. https://doi.org/10.1145/3097983.3098086.

The feedback management unit 130 maybe configured to perform continuous analytics on mood predictions using user data from the user sensor 110 and/or mobile device usage to determine the current mood state of the user. The mood predictor unit 210 may transform a given mood algorithm's outcome in order to generate a semiquantitative variable of mood, which may be used to provide a more precise prediction of mood beyond the simple binary state of positive or negative. Mood may, for example, be classed on a continuum from very negative to very positive: -3 (very negative), -2, -1, 0(neutral), +1, +2, +3 (very positive). Initially, this may be performed using a basic heuristic algorithm, but may also be determined and dynamically updated using a machine-learning algorithm.

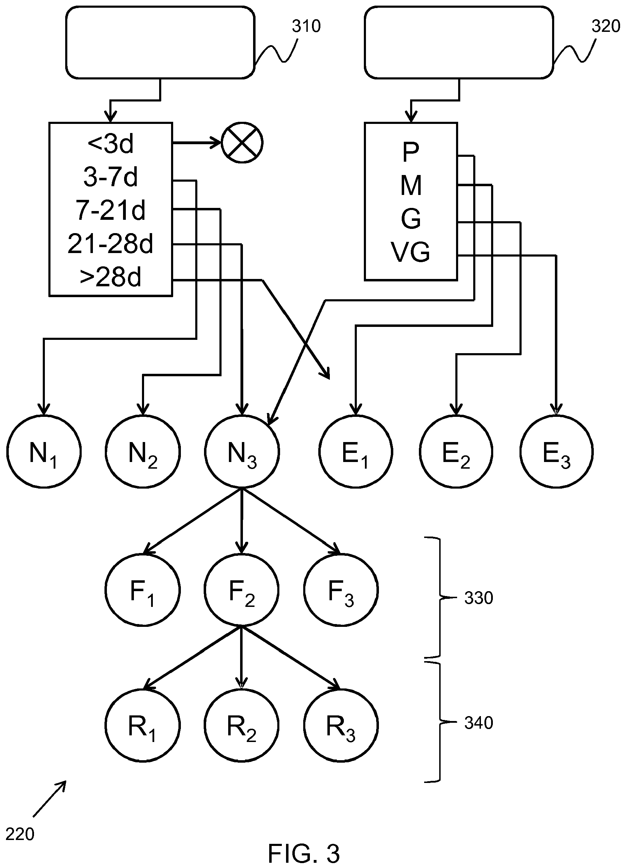

Further, feedback management unit 130 may comprise a stage and state detection module 220, which is adapted to obtain the user profile 225 (and may also receive historic user data from a memory unit 125) in order to determine a user experience level. The operation of the stage and state detection module 220 is discussed further below with reference to Figure 3.

As discussed above, the user experience level may be used to select the feedback type and the feedback valance by a feedback selection unit 230. The user mood prediction may then be combined with the selected feedback type and the feedback valance as inputs into a feedback trigger unit 240. The feedback selection unit may use the user experience level to alter the wording and/or appearance of the feedback provided to the user.

The feedback trigger unit 240 may be primed daily, or at any other suitable interval, with the most recently updated feedback type and valence data from the feedback selection unit 230. When the appropriate mood state to the selected feedback type and feedback valance, as determined by the mood predictor unit 210, is received then the feedback release may be triggered and the feedback presented to the user.

In other words, the feedback trigger unit 240 receives input from the mood predictor unit and from the feedback selection unit. Only when the correct conditions are met according to user mood and experience level, will the personalized feedback message created in the feedback selector unit be sent to the user. The feedback trigger unit may perform continuous analysis on the mood prediction of the user, whilst also assessing the historical pattern of recent mood predictions to ensure accurate mood detection. This may, for example, be performed through the assessment of recent data patterns using basic outlier detection methods, such as the Grubbs test method. If a given set of mood data is determined as an outlier, it may be excluded from the historical user data. A classical Bayesian prediction, or similar approach taking into account historical user data, may be employed to determine the probability of a mood prediction being correct based on past mood history. The most probable mood state may be selected based on the largest probability value obtained from a series of such predictions.

Figure 3 shows a schematic representation of an exemplary state and stage detector 220 of the feedback module 130.

The state and stage detector 220 may operate according to the following rule set; however, after significant user data aggregation, the rules may be governed by a machine learning algorithm trained using the available user data.

The state and stage detector 220 shown in Figure 3 takes the amount of time that the user has been pursuing the user goal 310 and the user's success in pursuing the goal 320 into account in order to generate a measure of the user's stage of progression towards the goal (novice: N₁; N₂; or N₃, or expert: E₁; E₂; or E₃) alongside self-efficacy 330 (F_{1,2, or 3}) and response efficacy 340 (R_{1,2, or 3}). The state and stage detector 220 shown in Figure 3 is adapted to generate tertiles (3 parts) from the continuous variables obtained from the user profile, which may correspond to early stage, mid stage, or late stage for the novice and expert categories and to high, medium, and low in the case of self-efficacy and response efficacy.

Data relating to the length of time that the user has been pursuing the goal may be analyzed first. These data may be obtained directly from the user profile. Four levels of time in program are used to assign a user to one of four categories: N₁, N₂, N₃, or Expert. In the example shown in Figure 3, if the time is less than three days (<3d) the user is not assigned a category. If the time is between three and seven days (3-7d), the user is assigned to the first novice category. If the time is between seven and twenty one days (7-21d), the user is assigned to the second novice category. If the time is between twenty one and twenty eight days (21-28d), the user is assigned to the third novice category. If the user has been pursuing the goal for more than twenty eight days (>28d), they maybe assigned to the expert category. It should be noted that, in the example shown in Figure 3, time spent pursuing the goal is not used to assign the user to a specific expert category, but as an indicator that the user is no longer a novice.

Data relating to the success of the user in pursuing the goal may be used to refine the designations of the expert categories. For example, if performance is poor (P), even at twenty eight days, then the individual may be designated as N₃ and remain in the novice category for the purposes of selecting the feedback type and valance.

According to the success level, users at greater than twenty eight days experience in pursuing the goal may be designated as E₁ (for moderate (M) performance), E₂ (for good (G) performance), or E₃ (for very good (VG) performance).

Following the designation of the user in one of the novice or expert categories, self-efficacy data 330 may be used to determine an user's level of self-efficacy from low (F₁), medium (F₂), or high (F₃). It will be appreciated that, although this stage is only shown for the N₃ category, this step may be implemented for any of the novice or expert categories. The user's self-efficacy score may be obtained by providing the user with a prompt, for example by way of the user interface using a dVAS, to provide feedback relating to how they perceive their progress towards their goal.

Finally, the user may be assigned a response-efficacy level 340 of R₁, R₂, or R₃ (corresponding to low, medium and high, respectively). Once again, it will be appreciated that, although this stage is only shown for the F₂ category, this step may be implemented for any of the self-efficacy categories of any of the novice or expert categories. The user's response efficacy score may be obtained by providing the user with a prompt, for example by way of the user interface, to provide feedback relating to how they perceive their progress towards their goal.

The user's self-efficacy and response efficacy scores may be obtained by way of an of electronic visual analogue scale (eVAS) or a dVAS by way of the user interface, which may collect user perceived self-efficacy and response efficacy data in a quantitative format, for example, in response to a number of questions. There may be three or less such questions for each of self-efficacy and response-efficacy score and the questions may be selected by experts in the goal domain according to established standards. The questions may be tailored according to the implementation of the system.

As such, a user, and more specifically the user's experience level, may be scored across three dimensions in order to arrive at an optimal selection of feedback valance and feedback type. In the example shown in Figure 3, 54 different user profiles are possible. For example, if a user were to receive a score of E₁F₃R₂, this user is an early expert (having a moderate level of success in pursuing the goal after more than twenty eight days) with a high self-efficacy score and a mid-level response efficacy score. In this case, this user may be provided with informative feedback with a negative valence while attempting to augment the lower level of response efficacy using a response efficacy boost.

For users with low self-efficacy (F₁) and/or low response efficacy (Ri) an additional boost may be provided with the feedback. For example, this boost may take the form of an encouraging message directed to enhance the user's confidence. The implementation of the boost may vary according to the implementation of the system.

Below is an example of how the systems described above maybe implemented.

The systems described above may be incorporated as an add-in module to a weight loss application (for example, on a smartphone or other smart device of a user), or may be included within the native application framework.

The user may be prompted to connect the application to available sensors within the smartphone and/or in a fitness tracker (or smartwatch) or any multitude of such devices. The system may be configured to synchronize with an existing application's goal setting and feedback features.

The stage and state detection module may collect user engagement data (such as time active in pursuing the goal) as well as success data from the application's existing tracking and/or monitoring features. These data may be used to maintain an ongoing assessment of the stage of the user. In addition, at pre-determined intervals (such as, biweekly, but this may be adjusted) the user's state (self-efficacy and response-efficacy scores) may be assessed using an eVAS questionnaire method and is completed by user manual input after an automated prompt is provided via a push notification.

These data may then be sent to the feedback type and valence selector module of the feedback management unit, which uses a rule set such as that described above to select the appropriate feedback content for the user's present stage and state.

Alongside the stage and state detection module, the mood predictor unit may be using user sensor input and algorithms to log a continuous assessment of the user's mood state over time. These mood state data may then be sent to the feedback trigger module at regular intervals.

When the selected feedback type and valence and the coincident mood state satisfy the trigger criteria, the feedback trigger module may provide the feedback to the user.

For example, a user who has recently started (novice) using a weight loss app or weight management program will typically receive only verification type feedback with a clear positive valence and only during times of positive mood (to encourage reinforcement learning) as they build their commitment towards the weight goal. This approach may enhance motivation and drive ongoing effort towards weight loss.

With passing time and a good record of success in achieving early goals and targets, the user may graduate to the expert stage. At this point, the feedback type may be largely informative, with different degrees of detail based on the user's Expertise level: 1, 2, or 3. This feedback may have a negative valence to better motivate the user at this stage. As the feedback has a negative valence, it should be delivered at a time of positive mood so that the cognitive resources are available to appropriately process the information and ensure that the details of the gaps and actions needed are internalized fully and motivation is enhanced.

This is achieved by controlling the trigger for providing the correct type of feedback based on the mood prediction of the user.

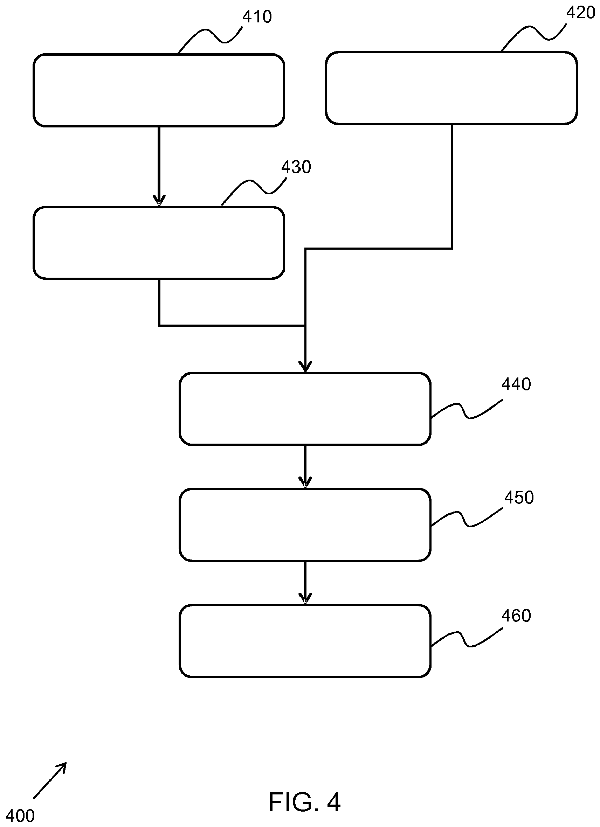

Figure 4 shows a method 400 for generating user specific feedback for a user.

The method begins by obtaining 410 user data, for example by way of a user sensor located in a smart device of the user, and obtaining 420 a user profile, the user profile comprising a user goal and a user experience level relating to the amount of time the user has been pursuing the user goal.

A mood prediction is then generated 430 based on the acquired user data and a feedback type and a feedback valance selected 440 based on the goal stage and the mood prediction.

Feedback is generated 450) based on the selected feedback type and feedback valance and the generated feedback is provided 460 to the user.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A system (100) for generating user-based feedback for a user, the system comprising:
a user sensor (110) adapted to acquire user data;
a feedback management unit (130), wherein the feedback management unit is adapted to:
obtain a user profile, the user profile comprising:
a user goal; and
a user experience level relating to the amount of time the user has been pursuing the user goal;
generate a mood prediction based on the acquired user data;
select a feedback type and a feedback valance based on the user experience level and the mood prediction; and
generate feedback based on the selected feedback type and feedback valance; and
a user interface (140) adapted to provide the generated feedback to the user.

2. A system (100) as claimed in claim 1, wherein the user sensor is adapted to sense one or more of:
a movement pattern;
a physical activity;
a galvanic skin response;
a heart rate; and
a user interaction with a system.

3. A system (100) as claimed any of claims 1 to 2, wherein the user profile further comprises historic user data.

4. A system (100) as claimed in claim 3, wherein the feedback management unit (130) is adapted to generate a mood prediction comprises comparing the obtained user data to the historic user data.

5. A system (100) as claimed in any of claims 1 to 4, wherein the user profile further comprises a goal performance relating to an attainment of the user goal, and wherein the feedback management unit (130) is further adapted to select the feedback type and the feedback valence based on the goal performance.

6. A system (100) as claimed in any of claims 1 to 5, wherein the user profile further comprises a user self-assessment relating to a user's perception of their performance towards the user goal, and wherein the feedback management unit (130) is further adapted to select feedback type and the feedback valence is further based on the user self-assessment.

7. A system (100) as claimed in any of claims 1 to 6, wherein the feedback type comprises one or more of:
verification feedback; and
informative feedback.

8. A system (100) as claimed in any of claims 1 to 7, wherein the feedback valance comprises one or more of:
positive feedback; and
negative feedback.

9. A system (100) as claimed in any of claims 1 to 8, wherein the user sensor (110) and the user interface (140) are located within a smart device of the user.

10. A system (100) as claimed in claim 9, wherein the smart device comprises one or more of:
a smartphone; and
a smart watch.

11. A computer-implemented method (400) for generating user specific feedback for a user, the method comprising:
obtaining (410) user data;
obtaining (420) a user profile, the user profile comprising:
a user goal; and
a user experience level relating to the amount of time the user has been pursuing the user goal;
generating (430) a mood prediction based on the acquired user data;
selecting (440) a feedback type and a feedback valance based on the user experience level and the mood prediction;
generating (450) feedback based on the selected feedback type and feedback valance; and
providing (460) the generated feedback to the user.

12. A method (400) as claimed in claim 11, wherein the user profile further comprises goal performance, and wherein selecting the feedback type and the feedback valence is further based on the goal performance.

13. A method (400) as claimed in any of claims 11 to 12, wherein the user profile further comprises a user self-assessment, and wherein selecting the feedback type and the feedback valence is further based on the user self-assessment.

14. A method (400) as claimed in any of claims 11 to 13, wherein the method further comprises updating the user profile based on the user data and the selected feedback type and feedback valance.

15. A computer program comprising computer program code means which is adapted, when said computer program is run on a computer, to implement the method of any of claims 11 to 14.
